# EUROPEAN PATENT APPLICATION

(11) **EP 4 360 620 A1**
(43) Date of publication of application: **01.05.2024**
(21) Application number: 23204947.8
(22) Date of filing: 20.10.2023
(51) Int. Cl.: A61K 9/00, A61K 9/08, A61K 31/4015, A61K 47/38, A61P 25/10

(54) **PEDIATRIC ORAL PHARMACEUTICAL SOLUTION OF ETHOSUXIMIDE**

(30) Priority: 21.10.2022 EP 22383021
(71) Applicant: Neuraxpharm Pharmaceuticals, S.L., 08970 Sant Joan Despí Barcelona (ES)
(72) Inventor: RIDHURKAR, Devendra, 08970 SANT JOAN DESPÍ (ES); UBEDA PEREZ, Carmen, 08970 SANT JOAN DESPÍ (ES); DIEZ MARTIN, Ignacio, 08970 SANT JOAN DESPÍ (ES)
(74) Representative: ZBM Patents - Zea, Barlocci & Markvardsen

(57) **Abstract**

The present invention provides an oral pharmaceutical solution comprising ethosuximide, a cellulose derivative, preferably hydroxypropyl methylcellulose, and one or more pharmaceutically acceptable aqueous excipients or additives, wherein the oral pharmaceutical solution is free of any preservative. The invention also relates to a process for preparing said oral pharmaceutical solution and its use in the treatment of epilepsy.

## Description

### FIELD OF THE INVENTION

The present invention relates to stable oral pharmaceutical solutions of ethosuximide free of any preservative, with good palatability, particularly beneficial for the pediatric patients. The invention also relates to a process for preparing said oral pharmaceutical solution and its use in the treatment of epilepsy.

### BACKGROUND OF THE INVENTION

3-Ethyl-3-methyl-2,5-pyrrolidinedione, also known as ethosuximide, compound of formula (I), is an anticonvulsant of the class of succinimides that apparently exerts multiple mechanisms of action.

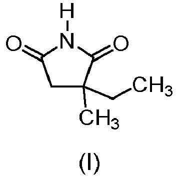

Ethosuximide is indicated for the treatment of epilepsy, pyknoleptic absences, as well as complex and atypical absences epilepsy. Myoclonic-astatic petit mal and myoclonic fits of adolescents (impulsive petit mal) if other medicines are not effective and/or are not tolerated. Ethosuximide is commercialized in the form of soft gelatin capsules and in oral solution (syrup).

When administered in form of soft gelatin capsules or oral solutions, such dosage forms generally contain water. There is a risk of microbial growth in these oral pharmaceutical formulations due to the presence of water. To overcome this problem preservatives are commonly used.

US2993835 A discloses pharmaceutical compositions of ethosuximide for the treatment of epilepsy. Particularly, an aqueous solution of ethosuximide is prepared in example 5 containing sucrose, sorbitol as co-solvent, oil of orange and sodium benzoate as preservative. The oral solution of ethosuximide prepared in a concentration of 25 mg/mL is filled into bottles each containing 100 ml.

Zarontin syrup contains glycerol as co-solvent, citrate buffer, sucrose, glucose, saccharin sodium, raspberry flavor, sodium benzoate as preservative, and purified water. Each 5 mL for oral administration contains 250 mg of ethosuximide (i.e., 250 mg/5mL).

Therefore, from what is known in the art, it is derived that there is still the need of providing stable oral pharmaceutical solutions of ethosuximide that avoid not only preservatives such as sodium benzoate and parabens, but also co-solvents such as glycerol, which would be especially beneficial for pediatric patients.

### SUMMARY OF THE INVENTION

The present inventors have surprisingly found that the oral pharmaceutical aqueous solution of ethosuximide containing a cellulose derivative, preferably hypromellose (HPMC), and one or more pharmaceutically acceptable aqueous excipients or additives, does not require the presence of any preservative to have good stability not only under long term but also under accelerated conditions upon storage.

Therefore, a first aspect of the present invention relates to an oral pharmaceutical solution comprising ethosuximide, a cellulose derivative, preferably hypromellose (hydroxypropyl methylcellulose, HPMC), and one or more pharmaceutically acceptable aqueous excipients or additives, wherein the oral pharmaceutical solution is free of any preservative.

A second aspect of the invention provides a process for preparing the oral pharmaceutical solution as defined above, comprising the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii).

A third aspect of the invention refers to the oral pharmaceutical solution as defined above for use in the treatment of epilepsy.

### DETAILED DESCRIPTION OF THE INVENTION

According to the first aspect of the present invention, the oral pharmaceutical solution comprises:
a) ethosuximide,
b) a cellulose derivative, preferably hypromellose (HPMC), and
c) one or more pharmaceutically acceptable aqueous excipients or additives,
wherein the oral pharmaceutical solution is free of any preservative.

The solvent of the oral pharmaceutical solution of the present invention is water. In a particular embodiment of the first aspect, the oral pharmaceutical solution of ethosuximide is free of any co-solvent such as alcohol, glycerol and propylene glycol.

In another particular embodiment, ethosuximide, as an active ingredient, is present in the oral pharmaceutical solution in an amount of 25 mg, 50 mg, 100 mg, and 150 mg per mL of the oral pharmaceutical solution. In other words, ethosuximide is present in a concentration of from 25 mg/ml to 150 mg/ml, preferably from 50 mg/ml to 100 mg/ml, more preferably ethosuximide is present in the oral pharmaceutical solution in a concentration of 25 mg/mL, 50 mg/mL and 100 mg/ml, even more preferably in 100 mg/mL since a relatively small volume to swallow is needed which is beneficial for pediatric patient in treatment compliance.

In another particular embodiment, the oral pharmaceutical solution comprises:
a) ethosuximide in an amount of 100 mg per mL of the oral pharmaceutical solution
b) a cellulose derivative, preferably hypromellose (HPMC), and
c) one or more pharmaceutically acceptable aqueous excipients or additives,
wherein the oral pharmaceutical solution is free of any preservative.

The cellulose derivative refers to methyl cellulose, hydroxypropyl cellulose, hydroxypropyl methylcellulose (hypromellose or HPMC); pregelatinized starch, or mixtures thereof. Preferably, the cellulose derivative is selected from the group consisting of methyl cellulose, hydroxypropyl cellulose, hydroxypropyl methylcellulose and mixtures thereof. More preferably, the cellulose derivative is hydroxypropyl methylcellulose. Suitable available hypromellose is selected from but not limited to Benecel K250 PH, Methocel K100 LV, Methocel E50 LV, Methocel E15 LV, and Pharmacoat 615.

In another particular embodiment, the oral pharmaceutical solution comprises:
a) ethosuximide,
b) a cellulose derivative selected from the group consisting of methyl cellulose, hydroxypropyl cellulose, hydroxypropyl methylcellulose and mixtures thereof, preferably hypromellose (HPMC), and
c) one or more pharmaceutically acceptable aqueous excipients or additives,
wherein the oral pharmaceutical solution is free of any preservative.

In another particular embodiment, the oral pharmaceutical solution comprises ethosuximide, hydroxypropyl methylcellulose (hypromellose) and one or more pharmaceutically acceptable aqueous excipients or additives, wherein the oral pharmaceutical solution is free of any preservative.

In another particular embodiment, the cellulose derivative, preferably hypromellose, has a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C.

In another particular embodiment, the oral pharmaceutical solution comprises:
a) ethosuximide,
b) hydroxypropyl methylcellulose (hypromellose) having a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C, and
c) one or more pharmaceutically acceptable aqueous excipients or additives,
wherein the oral pharmaceutical solution is free of any preservative.

In another particular embodiment, the amount of the cellulose derivative, preferably hypromellose, in the oral pharmaceutical solution is of from 3% to 5% by weight of the total weight of the pharmaceutical solution, preferably is 3.3% by weight of the total weight of the pharmaceutical solution.

In another embodiment, the oral pharmaceutical solution comprises:
a) ethosuximide,
b) the cellulose derivative, preferably hypromellose, in an amount of 3% to 5% by weight of the total weight of the pharmaceutical solution, preferably in an amount of 3.3%, and
c) one or more pharmaceutically acceptable aqueous excipients or additives,
wherein the oral pharmaceutical solution is free of any preservative.

In another embodiment, the oral pharmaceutical solution comprises:
a) ethosuximide,
b) a cellulose derivative, preferably hypromellose, having a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C and wherein the amount of the cellulose derivative, preferably hypromellose, in the oral pharmaceutical solution is of from 3% to 5% by weight of the total weight of the pharmaceutical solution.
c) and one or more pharmaceutically acceptable aqueous excipients or additives,
wherein the oral pharmaceutical solution is free of any preservative,

In another embodiment of the first aspect, the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C.

In another embodiment, the oral pharmaceutical solution comprises:
a) ethosuximide,
b) a cellulose derivative, preferably hypromellose (HPMC), and
c) one or more pharmaceutically acceptable aqueous excipients or additives,

wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C, and
wherein the oral pharmaceutical solution is free of any preservative.

In another embodiment, the oral pharmaceutical solution comprises:
a) ethosuximide,
b) a cellulose derivative, preferably hypromellose, having a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C,
c) and one or more pharmaceutically acceptable aqueous excipients or additives,

wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C, and
wherein the oral pharmaceutical solution is free of any preservative.

In another embodiment, the oral pharmaceutical solution comprises:
a) ethosuximide,
b) a cellulose derivative, preferably hypromellose, having a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C, and
   wherein the amount of the cellulose derivative, preferably hypromellose, in the oral pharmaceutical solution is of from 3% to 5% by weight of the total weight of the pharmaceutical solution, and
c) one or more pharmaceutically acceptable aqueous excipients or additives,

wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C, and
wherein the oral pharmaceutical solution is free of any preservative,

In another embodiment, the oral pharmaceutical solution comprises:
a) ethosuximide in an amount of 100 mg per mL of the oral pharmaceutical solution,
b) a cellulose derivative, preferably hypromellose, having a viscosity of from 10 mPa*s to 120 mPa*s, as measured in a 2% weight/volume aqueous solution of said cellulose derivative at 20°C,
c) and one or more pharmaceutically acceptable aqueous excipients or additives,

wherein the amount of the cellulose derivative, preferably hypromellose, in the oral pharmaceutical solution is of from 3% to 5% by weight of the total weight of the pharmaceutical solution,
wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C, and
wherein the oral pharmaceutical solution is free of any preservative,

The preservative refers to an agent or mixture of agents that is used to protect a composition against antimicrobial (e.g., yeast, mold, bacteria) activity. Representative preservatives include, but are not limited to, sodium benzoate, benzoic acid, ethylenediaminetetraacetic acid, sorbic acid, benzethonium chloride, benzalkonium chloride, bronopol, butyl paraben, methyl paraben, ethyl paraben, propyl paraben, thiomersal, sodium propionate, chlorhexidine, chlorobutanol, chlorocresol, cresol, imidurea, phenol, phenylmercuric salts, potassium sorbate, and mixtures thereof.

In another embodiment, the preservative of which the solution is free is selected from the group consisting of alcohol, ethanol, chlorobutanol, phenoxyethanol, potassium benzoate, benzyl alcohol, benzoic acid, potassium sorbate, sorbic acid, benzalkonium chloride, cetrimonium bromide, cetylpyridinium chloride, bronopol, chlorbutol, chlorocresol, cresol, butylparaben, methylparaben (or methyl-4-hydroxybenzoate), propylparaben, ethylparaben, phenol, thymol, phenylethanol, and sodium benzoate.

In another embodiment, the one or more pharmaceutically acceptable aqueous excipients or additives are selected from the group comprising of solubilizers, solubility enhancing agents, suspending agents/thickening agents/viscosity modifying agents, permeation/penetration enhancers, tonicity agents, mucoadhesives, bulking agents/auxiliary suspending agents, chelating agents, wetting agents, antifoaming agents, anti-caking agents, stabilizing agents, buffering agents and/or pH modifying agents and/or pH adjusting agents, surfactants, sweetening agents, flavoring agents, taste boosters, coloring agents, and combination thereof.

The buffering agent refers to an agent or a mixture of agents that can maintain the original acidity or basicity of a composition. Representative buffering agents include, but are not limited to, citric acid, sodium citrate, sodium phosphate, potassium citrate, and mixtures thereof. Preferably, the buffering agent is a citrate buffer. The buffering agent or mixtures thereof are present in an amount of from 0.01 % to 20% by weight of the oral pharmaceutical solution, preferably from 0.01 % to 10% by weight of the oral pharmaceutical solution, more preferably from 0.01 % to 2% by weight of the oral pharmaceutical solution.

In another embodiment, the pH of the oral pharmaceutical solution is of from 4.6 to 5.8, preferably of from 4.8 to 5.6.

In another embodiment, the oral pharmaceutical solution comprises:
a) ethosuximide,
b) a cellulose derivative, preferably hypromellose (HPMC), and
c) one or more pharmaceutically acceptable aqueous excipients or additives,

wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C,
wherein the pH of the oral pharmaceutical solution is of from 4.6 to 5.8, preferably of from 4.8 to 5.6, and
wherein the oral pharmaceutical solution is free of any preservative.

Taste is one of the most important parameters governing patient compliance. Undesirable taste is one of several important formulation problems that are encountered with certain drugs. Oral administration of bitter drugs with an acceptable degree of palatability is a key issue for health care providers, especially for pediatric patients. Taste masking with flavors and sweeteners technique is the foremost and the simplest approach for taste masking, especially in the case of pediatric formulations, chewable tablets, and liquid formulations. Artificial sweeteners and flavors are generally being used for taste-masking to improve the taste of oral solutions. Artificial flavors include any substance, the function of which is to impart flavor, which is not derived from a spice, fruit or fruit juice, vegetable, or vegetable juice.

The sweetener agent refers to an agent to mask the bitter taste of the active ingredient, i.e., ethosuximide, or to impart sweetness to the oral pharmaceutical solution. Suitable sweeteners are selected from sugar such as monosaccharide or disaccharides, for example D-glucose, D-fructose, D-xylose, maltose or sucrose; dulcitol, mannitol, sorbitol or xylitol, or artificial sweeteners, such as saccharin or the corresponding sodium, potassium or calcium salt, cyclamate or the corresponding sodium or calcium salt, aspartame, or acesulfame or the potassium salt thereof, furthermore Dulcin or ammonium glycyrrhizinate, or mixtures thereof. Preferably, the sweeting agent is sodium saccharin. The sweetening agent or mixtures thereof are present in an amount of from 0.001 % to 20% by weight of the oral pharmaceutical solution, preferably in an amount of from 0.01 % to 20% by weight of the oral pharmaceutical solution, more preferably from 0.01 % to 2% by weight of the oral pharmaceutical solution, even more preferably in an amount of 0.8% by weight of the oral pharmaceutical solution.

Suitable flavoring agents may be used in the oral pharmaceutical solution such as cherry flavor, strawberry flavor, caramel flavor or other suitable flavor to make the solution easier for a patient to ingest. Preferably, the flavoring agent is strawberry flavor. The flavoring agent or mixtures thereof are present in an amount of from 0.0001 % to 5% by weight of the oral pharmaceutical solution, preferably of from 0.01% to 2% by weight of the oral pharmaceutical solution.

Sweet booster is a very light brown, clear to slightly cloudy liquid with characteristic smell and taste. It reduces or inhibits the bitterness of drugs without affecting the other taste modalities such as sweetness or saltiness.

Advantageously, the combination of the sweet booster and the strawberry flavor increases the palatability of the oral pharmaceutical solution suitable for the compliance in pediatric patients even with a concentrated solution of 100 mg/ml.

In another embodiment, the one or more pharmaceutically acceptable aqueous excipients or additives are selected from the group comprising of a buffering agent, a pH modifying agent, a pH adjusting agent, a sweetening agent, a flavoring agent, a taste booster, a coloring agent, and mixtures thereof.

In another embodiment, the oral pharmaceutical solution comprises:
a) ethosuximide,
b) a cellulose derivative, preferably hypromellose (HPMC), and
c) one or more pharmaceutically acceptable aqueous excipients or additives selected from the group comprising of a buffering agent, a pH modifying agent, a pH adjusting agent, a sweetening agent, a flavoring agent, a taste booster, a coloring agent, and mixtures thereof,
wherein the oral pharmaceutical solution is free of any preservative.

In another embodiment, the oral pharmaceutical solution comprises:
a) 5-20% by weight, preferably 10% by weight, of ethosuximide
b) 3-5% by weight of a cellulose derivative, preferably hypromellose (HPMC), and
c) one or more pharmaceutically acceptable aqueous excipients or additives selected from the group comprising of a buffering agent, a pH modifying agent, a pH adjusting agent, a sweetening agent, a flavoring agent, a taste booster, a coloring agent, and mixtures thereof,
wherein the oral pharmaceutical solution is free of any preservative, and wherein all the percentages are in weight with respect to the total weight of the pharmaceutical solution.

In another embodiment, the oral pharmaceutical solution comprises:
a) 5-20% by weight, preferably 10% by weight, of ethosuximide
b) 3-5% by weight of a cellulose derivative, preferably hypromellose (HPMC), and
c) 0.01 % to 2% by weight of a buffering agent, a pH modifying agent or a pH adjusting agent,
d) 0.01% to 2% by weight, preferably 0.8% by weight, of a sweetening agent,
e) 0.01% to 2% by weight, preferably 0.2% by weight, of a flavoring agent,
f) 0.01% to 2% by weight, preferably 0.2% by weight, of a taste booster, and
wherein the oral pharmaceutical solution is free of any preservative, and wherein all the percentages are in weight with respect to the total weight of the pharmaceutical solution.

In another embodiment, the oral pharmaceutical solution comprising ethosuximide, a cellulose derivative, preferably hypromellose (HPMC), and one or more pharmaceutically acceptable aqueous excipients or additives, wherein the oral pharmaceutical solution is free of any preservative and wherein the oral pharmaceutical solution is free of any co-solvent.

The co-solvent refers to an organic molecule capable of at least partially dissolving another substance (i.e., the solute) selected from the group consisting of glycerol, propylene glycol, and low molecular weight polyethylene glycol up to 400 average molecular weight, and a mixture thereof. Polyethylene glycol known also as macrogol, are polymers of ethylene oxide with the following chemical structure: HOCH₂-[CH₂-CH₂-O]ₙ-CH₂OH (n: average number of ethylene oxide units). Within the pharmaceutical industry the number included in the names of polyethylene glycols indicates the average molecular weight. Polyethylene glycols with an average molecular weight equal to or lower than 600, preferably with an average molecular weight of equal to or up to 400, are non-volatile, clear, viscous, colorless or almost colorless hygroscopic liquids at 25°C, easily soluble in water in any ratio, even in hard water and in the presence of various salts, so especially suitable to be included in liquid formulations.

Additionally, propylene glycol and its acidic metabolites may have negative side effects in children, particularly in children equal or below 5 years of age as stated in the report "Propylene glycol used as an excipient" published by European Medicines of Agency (EMA/CHMP/704195/2013). As stated in the report, the following adverse events have been linked to propylene glycol exposure in patients, when administered as an excipient with various medicinal products: hyperosmolality, lactic acidosis; renal dysfunction (acute tubular necrosis), acute renal failure; cardiotoxicity (arrhythmia, hypotension); central nervous system (depression, coma, seizures); respiratory depression, dyspnoea; liver dysfunction; haemolytic reaction (intravascular hemolysis) and haemoglobinuria; multisystem organ dysfunction.

In another embodiment, the oral pharmaceutical solution is free of any co-solvent selected from the group consisting of maltitol, glycerol, mannitol, sorbitol, xylitol, propylene glycol, low molecular weight polyethylene glycol with average molecular weight equal to or lower than 600, preferably of equal to or lower than 400 average molecular weight, and a mixture thereof. Advantageously, the oral pharmaceutical solution of the invention free of propylene glycol avoids the above potential adverse effects. Additionally, the oral pharmaceutical solution free of propylene glycol is clear and no precipitation is observed.

In another embodiment, the oral pharmaceutical solution comprises:
a) ethosuximide,
b) a cellulose derivative, preferably hydroxypropyl methylcellulose (hypromellose),
c) and one or more pharmaceutically acceptable aqueous excipients or additives,

wherein the oral pharmaceutical solution is free of any preservative, and
wherein the oral pharmaceutical solution is free of any co-solvent.

In another embodiment, the oral pharmaceutical solution comprises:
a) ethosuximide,
b) a cellulose derivative, preferably hydroxypropyl methylcellulose (hypromellose),
c) and one or more pharmaceutically acceptable aqueous excipients or additives,

wherein the oral pharmaceutical solution is free of any preservative selected from the group consisting of alcohol, ethanol, chlorobutanol, phenoxyethanol, potassium benzoate, benzyl alcohol, benzoic acid, potassium sorbate, sorbic acid, benzalkonium chloride, cetrimonium bromide, cetylpyridinium chloride, bronopol, chlorbutol, chlorocresol, cresol, butylparaben, methylparaben (or methyl-4-hydroxybenzoate), propylparaben, ethylparaben, phenol, thymol, phenylethanol, and sodium benzoate,
and wherein the oral pharmaceutical solution is free of any co-solvent.

In another embodiment, the oral pharmaceutical solution comprises:
a) ethosuximide,
b) a cellulose derivative, preferably hydroxypropyl methylcellulose (hypromellose),
c) and one or more pharmaceutically acceptable aqueous excipients or additives,
wherein the oral pharmaceutical solution is free of any preservative selected from the group consisting of alcohol, ethanol, chlorobutanol, phenoxyethanol, potassium benzoate, benzyl alcohol, benzoic acid, potassium sorbate, sorbic acid, benzalkonium chloride, cetrimonium bromide, cetylpyridinium chloride, bronopol, chlorbutol, chlorocresol, cresol, butylparaben, methylparaben (or methyl-4-hydroxybenzoate), propylparaben, ethylparaben, phenol, thymol, phenylethanol, and sodium benzoate, and wherein the oral pharmaceutical solution is free of any co-solvent selected from the group consisting of maltitol, glycerol, mannitol, sorbitol, xylitol, propylene glycol, low molecular weight polyethylene glycol with average molecular weight equal to or lower than 600, preferably of equal to or lower than 400 average molecular weight, and a mixture thereof.

In another embodiment, the oral pharmaceutical solution comprises:
a) ethosuximide,
b) a cellulose derivative, preferably hydroxypropyl methylcellulose (hypromellose),
c) and one or more pharmaceutically acceptable aqueous excipients or additives,

wherein the oral pharmaceutical solution is free of sodium benzoate, and
wherein the oral pharmaceutical solution is free of any propylene glycol, and low molecular weight polyethylene glycol with average molecular weight equal to or lower than 600, preferably of equal to or lower than 400 average molecular weight, and a mixture thereof.

The oral pharmaceutical solution of the invention is therefore free of sugar, lactose, gluten, macrogol, parabens, sulfite, and tartrazine.

According to the second aspect, the process for the preparation of the oral pharmaceutical solution according to the first aspect, comprises the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii).

In another embodiment, the one or more pharmaceutically acceptable aqueous excipients or additives of step (iii) are selected from the group consisting of a buffering agent, a pH modifying agent, a pH adjusting agent, a sweetening agent, a flavoring agent, a taste booster, a coloring agent, and mixtures thereof.

In another embodiment, the process for the preparation of the oral pharmaceutical solution according to the first aspect, comprises the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii),
wherein the cellulose derivative, preferably hypromellose, of step (ii) has a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C.

In another embodiment, the process for the preparation of the oral pharmaceutical solution according to the first aspect, comprises the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii),

wherein the cellulose derivative, preferably hypromellose, of step (ii) has a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C, and
wherein the amount of the cellulose derivative, preferably hypromellose, of step (ii) is of from 3% to 5% by weight of the total weight of the oral pharmaceutical solution.

In another embodiment, the process for the preparation of the oral pharmaceutical solution according to the first aspect, comprises the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii),

wherein the cellulose derivative, preferably hypromellose, of step (ii) has a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C,
wherein the amount of the cellulose derivative, preferably hypromellose, of step (ii) is of from 3% to 5% by weight of the total weight of the oral pharmaceutical solution, and
wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C.

In another embodiment, the process for the preparation of the oral pharmaceutical solution according to the first aspect, comprises the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii),

wherein the cellulose derivative, preferably hypromellose, of step (ii) has a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C,
wherein the amount of the cellulose derivative, preferably hypromellose, of step (ii) is of from 3% to 5% by weight of the total weight of the oral pharmaceutical solution,
wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C, and
wherein the concentration of ethosuximide in the oral pharmaceutical solution is of from 25 mg/mL to 150 mg/mL, preferably of from 50 mg/mL to 100 mg/mL, even more preferably is of 100 mg/mL.

In another embodiment, the process for the preparation of the oral pharmaceutical solution according to the first aspect, comprises the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii),

wherein the cellulose derivative, preferably hypromellose, of step (ii) has a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C,
wherein the amount of the cellulose derivative, preferably hypromellose, of step (ii) is of from 3% to 5% by weight of the total weight of the oral pharmaceutical solution,
wherein the viscosity of the oral pharmaceutical solution is of from 40 mPa*s to 100 mPa*s, preferably is from 50 mPa*s to 90 mPa*s, more preferably from 50 mPa*s to 70 mPa*s as measured in a 2% aqueous solution at 20°C, and
wherein the concentration of ethosuximide in the oral pharmaceutical solution is of from 25 mg/mL to 150 mg/mL, preferably of from 50 mg/mL to 100 mg/mL, even more preferably is of 100 mg/mL, and
wherein the one or more pharmaceutically acceptable aqueous excipients or additives of step (iii) are selected from the group comprising of a buffering agent, a pH modifying agent, a pH adjusting agent, a sweetening agent, a flavouring agent, a taste booster, a coloring agent, and mixtures thereof.

In another embodiment, purified water is added to the final solution to reach a desired final volume of the oral pharmaceutical solution.

In another embodiment, the bulk solution is filtered through a suitable filter into a suitable container.

In another embodiment, the oral pharmaceutical solution is packaged in Type III amber glass bottles.

According to the third aspect, the oral pharmaceutical solution of the first aspect is suitable for use in the treatment of epilepsy, in particular, for the treatment of pyknoleptic absences as well as complex and atypical absences and myoclonic-astatic petit mal and myoclonic fits of adolescents (impulsive petit mal) if other medicines are not effective and/or are not tolerated.

In the following, the present invention is further illustrated by examples. They should in no case be interpreted as a limitation of the scope of the invention as defined in the claims. Unless indicated otherwise, all indications of percentage are by weight and temperatures are in degrees Celsius.

### GENERAL METHODS

### HPLC analysis:

Column: Waters Atlantis dC18 3 µm 150 mm x 4.6 mm
Flow: 1.2 mL/min
Wavelength: 210 nm
Column Temperature: 30 °C
Injection volume: 20 µL
Mobile phase A: Ultrapure water / Acetonitrile / Acetic Acid (91/8.9/0.1) (v/v/v)
Mobile phase B: Acetonitrile / Acetic acid (99.9/0.1) (v/v)
Elution: Gradient (Table 1)

**Table 1**

| Time (min) | Mobile phase A (%) | Mobile phase B (%) |
|---|---|---|
| 0 | 100 | 0 |
| 16.8 | 100 | 0 |
| 17.5 | 50 | 50 |
| 20.5 | 50 | 50 |
| 21.0 | 100 | 0 |

Analysis time 26.5 min
Diluent: Ultrapure water / Acetonitrile / Acetic Acid (91/8.9/0.1) (v/v/v)

### Standard stock solution:

Ethosuximide (10 mg) and Impurity A (2(RS)-2-ethyl-2-methylsuccinic acid, 10 mg) reference standard are accurately weighted in a 10 mL volumetric flask and diluted with solvent. After diluting and mixing, the flask is made up to the mark with solvent and mixed well again.
Concentration: Ethosuximide 1.0 mg/mL (5 %); Impurity A 1.0 mg/mL (5 %)

### Related Substances Standard solution:

1.0 mL of the standard stock solution is diluted with solvent to 10 mL.
Concentration: Ethosuximide 0.1 mg/mL (0.5 %); Impurity A 0.1 mg/mL (0.5 %)

The pH value of the oral pharmaceutical solution was measured in a pH Meter Metrohm 691 phmeter.

### EXAMPLES

### Example 1: Laboratory batch of ethosuximide 100mg/mL oral solution

**Table 2**

| Components | Amount (mg/mL) | Function |
|---|---|---|
| Ethosuximide | 100.00 | Active substance |
| Hypromellose (Pharmacoat 615) | 33.00 | Viscosity enhancer |
| Sodium saccharin (40-80 mesh) | 8.00 | Sweetening agent |
| Sodium citrate dihydrate | 0.55 | Buffering agent |
| Citric acid monohydrate | 0.25 | Buffering agent |
| Strawberry flavour (24FR340) | 2.00 | Flavouring agent |
| Sweet booster (50SW10) | 2.00 | Taste Booster |
| Purified water | 871.2 | Aqueous solvent |
| Total weight (mg) | 1017.00 | |

### Manufacturing procedure:

1) Purified water was heated to approximately 75°C. Ethosuximide was dissolved under stirring in hot purified water for 15 minutes. Subsequently, the solution was cooled down to approximately 70°C.
2) Hypromellose was added to solution of step (1) at 70°C while stirring for 30 minutes. Afterwards, the solution was cooled down to room temperature and stirred until clear solution was observed (speed-500 rpm).
3) Sodium saccharin and sodium citrate dihydrate were added to the solution obtained in step (2) and the obtained solution was stirred for 30 minutes.
4) Citric acid monohydrate was added to purified water and dissolved. The obtained solution was added to the solution resulting from step (3) to adjust the pH value. Also, strawberry flavour (e.g., 24FR340 from Dallant S.A.) and sweet booster (e.g., 50SW10 from Dallant S.A.) were added under continuous stirring for 15 minutes.
5) Purified water was added to the final solution to reach a final volume of 1015 g and stirring for 30 min.
6) The resulting solution of step (5) was filtered through a filter (3 µm PP filter) into a suitable container (e.g., 200 ml, Type III amber glass bottles).
7) The pH, refractive index and the viscosity of this final solution of Ethosuximide was analysed.

### Example 2: Stability studies

Stability study of the oral pharmaceutical solution prepared in Ex.1 in accelerated conditions of 40°C±2°C/75%±5% RH (Table 3) and under long-term conditions at 25°C±2°C/60%±5% RH (Table 4) up to 6 months.

**Table 3**

| Test | Initial (t=0) | 2 months | 3 months | 6 months |
|---|---|---|---|---|
| Appearance | Clear, colourless to slightly yellow solution | Clear, colourless to slightly yellow solution | Clear, colourless to slightly yellow solution | Clear, colourless to slightly yellow solution |
| Odor | Like Strawberry | Like Strawberry | Like Strawberry | Like Strawberry |
| pH value | 5.17 | 5.12 | 5.13 | 5.06 |
| Assay Ethosuximide (%) | 102.2 | 103 | 102 | 102.5 |
| Impurity A (%) | < 0.1% | 0.2% | 0.28% | 0.55% |
| Unknown impurities, single (%) | < 0.1% | < 0.1% | < 0.1% | LT 0.1% |
| Unknown impurities, total (%) | < 0.1% | 0.2% | 0.3% | 0.3% |
| TAMC | LT 10 CFU/mL | NA | NA | LT 10 CFU/mL |
| TYMC | LT 10 CFU/mL | NA | NA | LT 10 CFU/mL |
| E. coli | Absent in 1 mL | NA | NA | Absent in 1 mL |

| | | | | |
|---|---|---|---|---|
| Impurity A = 2(RS)-2-Ethyl-2-methylsuccinic acid; LT = Less than; TAMC = Total aerobic microbial count; TYMC = Total yeast and moulds count; E. coli = Escherichia coli | | | | |

**Table 4**

| Test | Initial (t=0) | 2 months | 3 months | 6 months |
|---|---|---|---|---|
| Appearance | Clear, colourless to slightly yellow solution | Clear, colourless to slightly yellow solution | Clear, colourless to slightly yellow solution | Clear, colourless to slightly yellow solution |
| Odor | Like Strawberry | Like Strawberry | Like Strawberry | Like Strawberry |
| pH value | 5.17 | 5.15 | 5.18 | 5.16 |
| Assay Ethosuximide (%) | 102.2 | 102.8 | 102.6 | 102.5 |
| Impurity A (%) | < 0.1% | 0.05% | 0.05% | 0.11% |
| Unknown impurities, single (%) | < 0.1% | < 0.1% | < 0.1% | < 0.1% |
| Unknown impurities, total (%) | < 0.1% | 0.10% | 0.10% | 0.10% |

| | | | | |
|---|---|---|---|---|
| Impurity A = 2(RS)-2-Ethyl-2-methylsuccinic acid | | | | |

The oral pharmaceutical solution of ethosuximide of the present invention without preservative showed good stability as demonstrated from the results of tables 3 and 4. Additionally, the oral pharmaceutical solution prepared as in Ex.1 showed antimicrobial activity according to the European Pharmacopoeia 10th edition criteria (5.1.4), in oral formulations, as shown in Table 3.

### Example 3. Scale-up batch of ethosuximide 100mg/mL oral solution

**Table 5**

| Components | Amount (mg/ml) | Amount (%) | Batch Size (Kg/60 L) |
|---|---|---|---|
| Ethosuximide | 100.00 | 10.000 | 6.000 |
| Hypromellose (Pharmacoat 615) | 33.00 | 3.300 | 1.980 |
| Sodium saccharin | 8.00 | 0.800 | 0.480 |
| Sodium citrate dihydrate | 0.55 | 0.055 | 0.033 |
| Citric acid monohydrate | 0.25 | 0.025 | 0.015 |
| Strawberry flavour (24FR340) | 2.00 | 0.200 | 0.120 |
| Sweet booster (50SW10) | 2.00 | 0.200 | 0.120 |
| Purified water | qs. 1ml | qs. 100 ml | qs. 60.000 L |
| Final weight | | 100.00 | 60.000 L |

| | | | |
|---|---|---|---|
| Qs. = sufficient quantity | | | |

### Manufacturing procedure:

1) Purified water (50.00 Kg) was heated to approximately 70°C in EMMSA reactor. Ethosuximide was added while stirring and after addition continue stirring for 30 minutes at 70°C until clear solution was observed (speed-500 rpm).
2) Hypromellose was added to step 1 solution while stirring and after addition continue stirring for 30 min. Then continue the stirring and the solution was cooled down to room temperature and continue stirring until clear solution was observed (speed-500 rpm).
3) Sodium saccharine, and sodium citrate dihydrate was added to the solution of step (2) and continue stirring for 60-90 min.
4) Citric acid monohydrate was added to 1.0 kg of purified water and dissolved. The obtained solution was added to the solution resulting from step (3) to adjust the pH value.
5) Strawberry flavour and taste booster were added under continuous stirring. Rinse all the containers with 1.0 kg of water and add the rinsing water in the solution and stir the solution for 30-45 minutes.
6) Purified water was added to the final solution of step (5) to reach a final volume of 60.00 L (60.90 kg) and keep stirring for 30 minutes.
7) The bulk solution was filtered through a Sartorius Midicaps size 8, polypropylene 3 µm filter using 1.0-2.0 bar pressure into a suitable container. Unfiltered, and filtered solution (bulk, initial, middle, and final) were collected in a suitable stainless-steel container. Initial 2-3 L were discarded, and the filtered solution was collected.
8) Physical parameters such as pH, bulk density, refractive index, and the viscosity of this final unfiltered and filtered solution were analysed.
9) The resulting solution was filled into 200 ml of Type III amber glass bottles.

### Example 4: Physical parameters of scale-up batch

The physicochemical properties unfiltered, filtered, and packed solutions of scale-up batch prepared in Ex. 3 were analysed and the results are gathered in Table 6.

**Table 6**

| Parameters | Scale-up batch of Ex.3 | | | | |
|---|---|---|---|---|---|
| | Bulk-unfiltered | Bulk | Initial | Middle | Final |
| Appearance | Clear, colourless to slightly yellow solution | | | | |
| Odor | Like Strawberry | | | | |
| Viscosity (cps) | 57.7 | 56.4 | 55.9 | 54.1 | 55.7 |
| pH value | 5.23 | 5.24 | 5.23 | 5.23 | 5.22 |
| Relative density (gm/ml) | 1.0212 | 1.0194 | 1.021 | 1.0195 | 1.0212 |
| Refractive Index | 1.35405 | 1.35409 | 1.35405 | 1.35407 | 1.35409 |
| Assay ethosuximide (%) | 100.3 | 100.2 | 99.7 | 100.1 | 99.9 |
| Impurity A (%) | ≤ 0.1 | ≤ 0.1 | ≤ 0.1 | ≤ 0.1 | ≤ 0.1 |
| Unknown impurities, single (%) | ≤ 0.1 | ≤ 0.1 | ≤ 0.1 | ≤ 0.1 | ≤ 0.1 |
| Unknown impurities, total (%) | ≤ 0.1 | ≤ 0.1 | ≤ 0.1 | ≤ 0.1 | ≤ 0.1 |
| TAMC | NA | LT 10² CFU/ml | LT 10² CFU/ml | LT 10² CFU/ml | LT 10² CFU/ml |
| TYMC | NA | LT 10 CFU/ml | LT 10 CFU/ml | LT 10 CFU/ml | LT 10 CFU/ml |
| E. coli | NA | absent in 1 ml | absent in 1 ml | absent in 1 ml | absent in 1 ml |

| | | | | | |
|---|---|---|---|---|---|
| Impurity A = 2(RS)-2-Ethyl-2-methylsuccinic acid; LT = Less than; TAMC = Total aerobic microbial count; TYMC = Total yeast and moulds count; E. coli = Escherichia coli | | | | | |

The physicochemical properties of unfiltered, filtered, and packed solutions were good and within the specifications that complies with pharmaceutical standards. The scale-up batch also complies with the microbial attribute requirements of Ph. Eur. (5.1.4) for aqueous preparations for oral use as demonstrated in Table 6.

### Example 5. Bioequivalence study

The assessment of bioequivalence was based upon 90% confidence intervals for the ratio of the population geometric means (test/reference) for the parameters under consideration. This method was equivalent to two one-sided tests with the null hypothesis of bio-inequivalence at the 5% significance level.

The pharmacokinetic parameters under consideration were analysed using ANOVA. The data was transformed prior to analysis using a logarithmic transformation. A confidence interval for the difference between formulations (i.e., the scale-up batch according to Example 3 and Zarontin 250mg/5mL) on the log-transformed scale was obtained from the ANOVA model. This confidence interval was then back-transformed to obtain the desired confidence interval for the ratio on the original scale.

The data was processed by ANOVA using standard test factors:
1. Fixed factors-treatment, period, subject, sequence
2. Nested factor-Subjects (Seq)
3. Interactions-sequences (as the fixed factor) and Subjects (as the nested factor)

The test product was to be considered as bioequivalent to the reference product if: Cmax & AUC0→72 ratio: The 90% confidence interval for this measure lies within an acceptance range of 80.00% - 125.00%

**Table 7**

| | | | | |
|---|---|---|---|---|
| Bioequivalence Results Summary | Parameter | Point Estimate (Ratio of geometric mean %) | Lower limit % | Upper limit % |
| | AUC_{0→72} (µg*h/mL) | 95.47 | 93.24 | 97.75 |
| | Cₘₐₓ (µg/mL) | 94.22 | 86.59 | 102.52 |

As it is demonstrated in the table 7, the concentrated oral pharmaceutical solution (i.e., 100 mg/mL) is bioequivalent to reference oral solution Zarontin of 250 mg/5 ml.

## Claims

1. Oral pharmaceutical solution comprising ethosuximide, a cellulose derivative, preferably, hydroxypropyl methylcellulose, and one or more pharmaceutically acceptable aqueous excipients or additives, wherein the oral pharmaceutical solution is free of any preservative.

2. The oral pharmaceutical solution according to claim 1, wherein the ethosuximide is in a concentration from 25 mg/ml to 150 mg/ml, preferably from 50 mg/ml to 100 mg/ml, more preferably is 100 mg/ml.

3. The oral pharmaceutical solution according to any one of claims 1 to 2, wherein the cellulose derivative is selected from the group of methyl cellulose, hydroxypropyl cellulose, hydroxypropyl methylcellulose, pregelatinized starch, and mixtures thereof.

4. The oral pharmaceutical solution according to any one of claims 1 to 3, wherein the cellulose derivative, preferably hypromellose, has a viscosity of from 10 mPa*s to 500 mPa*s, preferably from 10 mPa*s to 300 mPa*s, more preferably from 10 mPa*s to 120 mPa*s, as measured in a 2% weight/volume aqueous solution of said cellulose derivative at 20°C.

5. The oral pharmaceutical solution according to any one of claims 1 to 4, wherein the amount of the cellulose derivative, preferably hypromellose, is of from 3% to 5%, preferably is 3.3 % by weight of the total weight of the pharmaceutical solution.

6. The oral pharmaceutical solution according to any one of claims 1 to 5, wherein one or more pharmaceutically acceptable aqueous excipients or additives are selected from the group consisting of a buffering agent, a pH modifying agent, a pH adjusting agent, a sweetening agent, a flavouring agent, a taste booster, a colouring agent, and mixtures of any of them.

7. The oral pharmaceutical solution according to claim 6, wherein the buffering agent is a citrate buffer.

8. The oral pharmaceutical solution according to claim 6, wherein the flavouring agent is strawberry flavour.

9. The oral pharmaceutical solution according to claim 6, wherein the taste booster is (50SW10) sweet booster.

10. The oral pharmaceutical solution according to any one of claims 1 to 9, wherein the viscosity of the solution is of from 40 mPa*s to 100 mPa*s, preferably is from 50 mPa*s to 90 mPa*s, more preferably from 50 mPa*s to 70 mPa*s as measured in a 2% aqueous solution at 20°C.

11. The oral pharmaceutical solution according to any one of claims 1 to 10, wherein the pH of the solution is of from 4.6 to 5.8, preferably of from 4.8 to 5.6.

12. The oral pharmaceutical solution according to any one of claims 1 to 11, wherein the preservative of which the solution is free is selected from the group consisting of alcohol, ethanol, chlorobutanol, phenoxyethanol, potassium benzoate, benzyl alcohol, benzoic acid, potassium sorbate, sorbic acid, benzalkonium chloride, cetrimonium bromide, cetylpyridinium chloride, bronopol, chlorbutol, chlorocresol, cresol, butylparaben, methylparaben (or methyl-4-hydroxybenzoate), propylparaben, ethylparaben, phenol, thymol, phenylethanol, and sodium benzoate.

13. The oral pharmaceutical solution according to any one of claims 1 to 12, wherein the solution is free of any co-solvent selected from the group consisting of maltitol, glycerol, mannitol, sorbitol, xylitol, propylene glycol, low molecular weight polyethylene glycol with average molecular weight equal to or lower than 600, preferably of equal to or lower than 400 average molecular weight, and a mixture thereof.

14. A process for the preparation of the oral pharmaceutical solution according to any of claims 1 to 13, comprising the following steps:
i. dissolving ethosuximide, in water at a temperature in the range from 35°C to 85°C, preferably 70°C,
ii. adding a cellulose derivative, preferably hypromellose, into the mixture obtained in step (i) and stirring until a solution is obtained at a temperature in the range from 35°C to 85°C, preferably 70°C,
iii. adding one or more pharmaceutically acceptable aqueous excipients or additives to the solution obtained in step (ii).

15. Oral pharmaceutical solution as defined in any one of claims 1 to 13, for use in the treatment of epilepsy.
